# EUROPEAN PATENT APPLICATION

(11) **EP 1 860 193 A1**
(43) Date of publication of application: **28.11.2007**
(21) Application number: 06010527.7
(22) Date of filing: 22.05.2006
(51) Int. Cl.: C12N 15/52, C12P 13/08, C12P 13/06

(54) **Promoter-activity modulation for branched-chain amino acid production**

(71) Applicant: Evonik Degussa GmbH, 40474 Düsseldorf (DE)
(72) Inventor: Elisakova, Veronika, 14000 Prague 4 (CZ); Holàtko, Jiri, 66902 Znojmo Czech Republic (CZ); Nesvera, Jan, 16000 Praha 6 (CZ); Prouza, Marek, 11000 Praha 10 (CZ); Pàtek, Miroslav., Dr, 16000 Praha 6 (CZ)

(57) **Abstract**

The present invention provides nucleotide sequences which can be used for construction of modified promoters of the genes involved in the valine, leucine and isoleucine biosynthesis pathways (ilvA, ilvB, ilvD, ilvE and leuA), the mutated promoters themselves, the vector for construction of the mutations in the chromosome, the strains carrying the modified promoters and a process for the fermentative production of branched-chain amino acids using these modified promoters in specific hosts in which genes are expressed from these promoters.

## Description

The present invention is directed to the production of branched-chain amino acids. In particular, the present invention deals with genetically modified organisms, which are capable of producing these amino acids by fermentation.

Amino acids produced by micro-organisms in industrial fermentation of cheap substrates like molasses, acetic acid and ethanol are used as feed and food additives in agriculture and food industry, as constituents of infusion solutions, as therapeutics in medicine and as reagents in chemical and pharmaceutical industry. The branched-chain amino acids (L-isoleucine, L-valine, L-leucine) are essential for human and animals and their production is therefore of great importance.

Amino acids are produced by various bacteria like Escherichia coli, Serratia marcescens and by a number of strains of coryneform bacteria (corynebacteria) such as Corynebacterium and Brevibacterium. Since corynebacteria have been shown to produce various amino acids efficiently, methods of random mutagenesis and selection are employed to improve the performance characteristics of the producing strains. In addition to wild-type strains, amino acids are thus produced by auxotrophic or bradytrophic mutants, regulatory mutants and combined (auxotrophic and regulatory) mutants.

Using chemical or physical mutagenesis, strains were obtained which are resistant to antimetabolites, such as an isoleucine analogue isoleucine hydroxyamate [Journal of Bacteriology 110:761-763 (1972)], the valine analogue 2-thiazolealanine [Agricultural and Biological Chemistry 39:1319-1322 (1975)] or the leucine analogue α-aminobutyrate [Bioscience Biotechnology Biochemistry 60:1386-1387 (1996)] or which are auxotrophic for regulatorily significant metabolites and produce e.g. branched-chain amino acids [Journal of General and Applied Microbiology 7: 52-69 (1961), Journal of General and Applied Microbiology 7: 41-51 (1961)].

Improvements of the process of amino acid production may relate also to measures concerning fermentation technology, for example stirring and oxygen supply, or to the composition of the nutrient media, such as for example sugar concentration during fermentation, or to separating and purifying the product by, for example, ion exchange chromatography, or to the intrinsic performance characteristics of the microorganism itself.

With the expansion of methods of recombinant DNA technology, the genetic techniques have also been used for improvement of strains of Corynebacterium, which produce branched-chain amino acids by amplifying individual biosynthesis genes for branched-chain amino acids and for investigating the effect on their production [For reviews on this subject see: Antonie van Leeuwenhoek 64:145-163 (1993), Amino Acids 6:281-272 (1994), Critical Reviews in Biotechnology 15:73-103 (1995), Annals of the New York Academy of Science 782:25-39 (1996), Journal of Biotechnology 56:167-182 (1997), Advances in Biochemical Engineering/Biotechnology 73:9-29 (2001), Advances in Biochemical Engineering/Biotechnology 79:59-112 (2003), Journal of Biotechnology 104:287-299 (2003)].

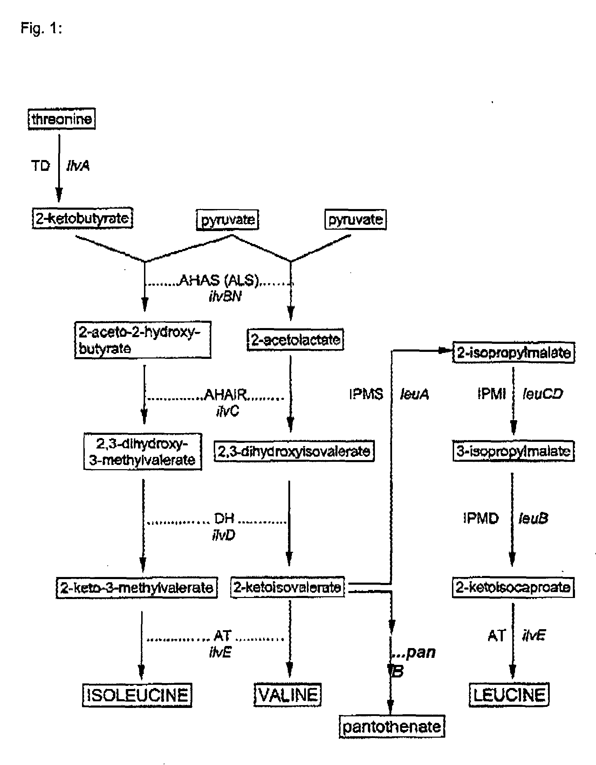

The biosynthesis of isoleucine, valine and leucine is closely connected. The scheme of biosynthesis of branched-chain amino acids in Corynebacterium glutamicum is shown in Fig. 1. In the parallel pathways, four enzymes (coded by the genes ilvBN, ilvC, ilvD and ilvE) catalyse the steps converting two molecules of pyruvate to valine and homologous steps converting 2-ketobutyrate and pyruvate to isoleucine. 2-ketobutyrate arises from threonine in a reaction catalysed by threonine dehydratase (coded by ilvA), specific for isoleucine pathway. Leucine-specific pathway starts from 2-ketoisovalerate, which is also the direct precursor of valine (Fig. 1).

Corynebacterium glutamicum producers of valine may be constructed by cloning the genes ilvBNC, ilvD and ilvE in a multicopy plasmid [(Applied and Environmental Microbiology 65:1973-1979 (1999), Applied and Environmental Microbiology 68:2246-2250 (2002)]. Deletions of the genes ilvA, panB and/or panC improve its production (EP1155139 B1). Valine yield may also be increased by using feedback-resistant mutants of acetohydroxy acid synthase (AHAS) in E. coli (Patent Application Publication US 2002/0037562 A1) and in C. glutamicum (Feedback Resistant Acetohydroxy Acid Synthetase Mutants. European Patent Application No. 03014640.1).

An alternative approach to adjust gene expression is to express the genes from chosen promoters of different strength. These promoters could be constitutive, inducible or synthetic. In another approach, native promoters may be altered by deletions, insertions and mutagenesis to modulate their activity. The structure of promoters of coryneform bacteria has been described [Microbiology 142:1297-1349 (1996), Journal of Biotechnology 104:311-323 (2003)]. Mutagenesis and analysis of the dapA promoter provided promoter variants showing a wide range of activities [Journal of Bacteriology 181:6188-6191 (1999)]. Mutations in the core -10 region have been studied and promoters with increased or decreased activity have been created [Journal of Bacteriology 181:6188-6191 (1999)]. Mutations, which strengthened the dapA promoter, were then introduced into the chromosome for development of C. glutamicum strains producing lysine (EP 1067193 A1, Advances in Biochemical Engineering/Biotechnology 73:9-29 (2001). This approach was used to construct a superior lysine producer.

The goal of present invention is to create a new way of how to produce branched-chain amino acids by micro-organisms more superior than known from the prior art. That is to say that micro-organisms are to be established which allow to generate said amino acids by fermentation in an advantageous manner from an economic and ecological point of view.

This objective is achieved according to a process embraced by instant claims. Claims 1 to 6 are directed to the process according to the invention. Claims 7 to 9 cover special promoters, recombinant DNA, plasmids, vectors, phages and micro-organisms. Claim 10 encompasses the use of present micro-organisms for the production of branched-chain amino acids.

By applying in a process for the production of branched-chain amino acids by fermentation a genetically modified bacterium of the genus of coryneform bacteria, like Corynebacterium or Brevibacterium, in which the promoter strength of one or more of the promoters selected from the group consisting of P-panX, P-ilvA and P-leuA is attenuated and the promoter strength of one or more of the promoters selected from the group consisting of P-ilvB, P-ilvC, P-ilvD and P-ilvE is increased, above-mentioned goal is arrived at in an easy but nonetheless surprising manner. The mere modulation of promoter strengths along the line of genes of the amino acid's biosynthetic pathway of the microorganism used lead to organisms being superior to those known in the art. This is even more surprising considering the fact that the biosynthesis of such amino acids is based on a complicated in vivo regulatory-mechanism on genetic level, which easily can be disturbed by interrupting or increasing the flow of each intermediate encountered during the bio-transformation focussed at.

According to the invention both branched-chain amino acids isoleucine and valine may be produced predominantly. However, by lowering the amount of 2-ketobutyrate present in the cell, the amount of valine prepared by fermentation may be further increased [Applied and Environmental Microbiology 65:1973-1979 (1999); Applied and Environmental Microbiology 68:2246-2250 (2202)]. Therefore, through a slight modification within the microorganism, valine is more efficiently produced by the process according to the invention.

Preferred micro-organisms, applied in the present process, are those which are derived from branched-chain amino acid-producing Corynebacterium or Brevibacterium. These micro-organisms are able to produce said amino acids from glucose, sucrose, lactose, mannose, fructose, maltose, molasses, starch, cellulose or from glycerol and ethanol. Within the genus Corynebacterium, the species Corynebacterium glutamicum may in particular be mentioned, which is known in specialist circles for its ability to produce enantiomerically enriched amino acids, preferably L-amino acids.

Suitable strains of the genus Corynebacterium, in particular of the species Corynebacterium glutamicum, are in particular the known wild type strains
Corynebacterium glutamicum ATCC13032
Brevibacterium flavum ATCC14067
Brevibacterium lactofermentum ATCC13869 and
Brevibacterium divaricatum ATCC14020
and branched-chain amino acid-producing mutants or strains produced therefrom,
such as for example the isoleucine producing strains
Corynebacterium glutamicum ATCC14309
Corynebacterium glutamicum ATCC14310
Corynebacterium glutamicum ATCC14311
Corynebacterium glutamicum ATCC15168
Corynebacterium ammoniagenes ATCC 6871,
such as for example the leucine producing strains
Corynebacterium glutamicum ATCC 21885
Brevibacterium flavum ATCC 21889
or such as for example the valine producing strains
Corynebacterium glutamicum DSM 12455
Corynebacterium glutamicum FERM-P 9325
Brevibacterium lactofermentum FERM-P 9324
Brevibacterium lactofermentum FERM-BP 1763.

Most preferably micro-organisms are used for present process which are composed of a genetically modified bacterium being derived from a bacterium selected from the group consisting of ATCC13032 and genetically related organisms like mutants of ATCC13032, for example the feedback resistant mutants M13 described in European patent application EP03014640.1.

P-panX denotes promoters for the genes involved in the bio-transformation of 2-ketoisovalerate to pantothenate in coryneform bacteria (Fig. 1). Preferably in a process according to the invention promoters of panB- and/or panC-genes are attenuated to support the flux of intermediates towards valine production by lowering the overall activity for the pantothenate synthesis.

Promoters of the genes which code for the enzymes of the pathways competing with the valine or isoleucine synthesis and draining off the valine precursors may be attenuated. By this approach, for example the promoter strength of P-leuA and/or P-ilvA is attenuated for higher isoleucine and/or valine production.

Promoters serve to regulate the gene expression in organisms. In micro-organisms these promoters can be identified according to the skilled worker's knowledge [Microbiolology 142: 1297-1309 (1996); Journal of Biotechnology 104:325-34 (2003)]. For example, special core sequences of these promoters, known as -10 and -35 hexamers, are located somewhat upstream the transcriptional start point of each gene. Preferably the extended -10 hexamers (-10 region) are to be the modified to regulate the respective gene expression. Especially preferred is a process wherein the sense - 10 promoter regions used for
a) ilvA are selected from the group consisting
   of CACAGT, CACTGT;
b) ilvD are selected from the group consisting
   of CACTGTGCTATAGTGTAAT, CAAAGCACTGTGGTATAAT;
c) ilvE are selected from the group consisting
   of TGTGGTACAAT, TGTGGTACCAT
d) leuA are selected from the group consisting
   of CATGCT, CAGGCT, CTGGCT, TATGCA, GAATGCA.

In another embodiment the present invention is directed to promoters for genes of coryneform bacteria, like Corynebacterium or Brevibacterium, selected from the group consisting of ilvA, leuA, ilvB, ilvD and ilvE comprising respective -10-motifs depicted above. Included are also the respective antisense sequences.

A further embodiment of the present invention is a recombinant DNA comprising instant promoter sequences and the respective genes selected from the group consisting of ilvA, leuA, ilvB, ilvD and ilvE.

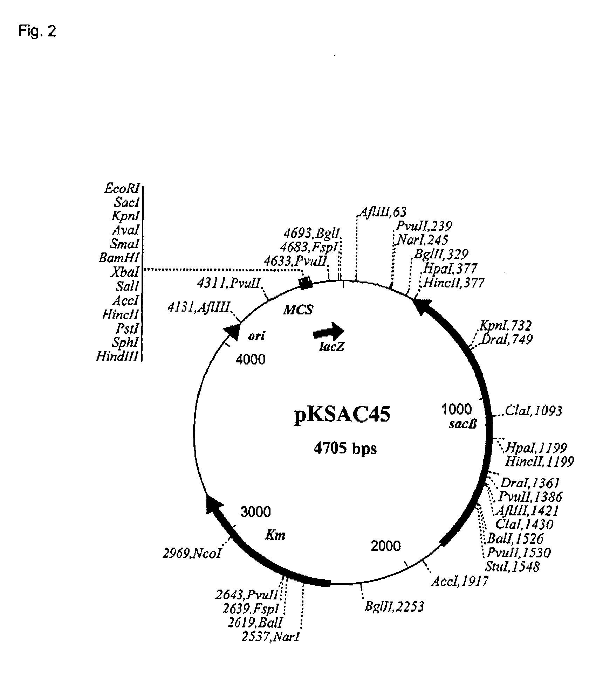

Still another embodiment is directed to micro-organisms, plasmids, vectors and phages comprising the recombinant DNA just mentioned.
In principle, suitable phages, plasmids or vectors are any of the variants available for this purpose to those skilled in the art. Such entities can be found in Studier et al., Methods Enzymol. 1990, 185, 61-69, or in the brochures issued by Roche Biochemicals, Invitrogen, Novagen, Promega, New England Biolabs, Clontech or Gibco BRL, Particularly preferred plasmids and vectors can be found in DNA cloning: a practical approach, Volume I-III, edited by D.M. Glover, IRL Press Ltd., Oxford, Washington DC, 1985, 1987; Denhardt, D.T. and Colasanti, J.: A survey of vectors for regulating expression of cloned DNA in E. coli. In: Rodriguez, R.L. and Denhardt, D.T. (eds). Vectors, Butterworth, Stoneham, MA, 1987, pp. 179-204; Gene expression technology. In: Goeddel, D.V. (eds), Methods in Enzymology, Volume 185, Academic Press, Inc., San Diego, 1990; Sambrook, J., Fritsch, E.F. and Maniatis. T. 1989. Molecular cloning: a laboratory manual, 2nd ed. Cold Spring Harbor Laboratory Press, Cold Spring Harbor, N.Y.
Plasmids with which the gene construct containing the nucleic acid according to the invention can very particularly preferably be cloned into the host organism are: pKK-177-3H (Roche Biochemicals), pBTac (Roche Biochemicals), pKK-233-3 (Amersham Pharmacia Biotech), pLex (Invitrogen) or the vectors of the pET (Novagen) or pUC-series like pUC18 or pUC19, respectively.
Especially preferred is a plasmid pKSAC45 according to Fig. 2. This plasmid is a new E. coli vector, which is easily transferred to Corynebacterium glutamicum by electrotransformation [FEMS Microbiology Letters 53:299-303 (1989); see Example 1].

Micro-organisms being preferably used for performing the process of the present invention are already discussed above. Other micro-organisms which are preferably applied in instant invention, for example to amplify the recombinant DNA, are known to the skilled worker. Micro-organisms which may be referred to in this respect are e.g. yeasts such as Hansenula polymorpha, Pichia sp., Saccharomyces cerevisiae, prokaryotes, E. coli, Bacillus subtilis or eukaryota, such as mammal cells, insect cells. Strains of E. coli are preferably used for this purpose. The following are very particularly preferred: E. coli XL1 Blue, NM 522, JM101, JM109, JM105, RR1, DH5α, TOP 10- or HB101. Also the processes used for this purpose are well-known to a person skilled in the art (Sambrook, J.; Fritsch, E. F. and Maniatis, T. (1989), Molecular cloning: a laboratory manual, 2nd ed., Cold Spring Harbor Laboratory Press, New York).

In a last embodiment present invention relates to the use of a microorganism as described above for the fermentative production of branched-chain amino acids.

Amino acids are often produced under the conditions of growth limitation brought about by shortage of some essential substrate [Applied Microbiology and Biotechnology 38:259-262 (1992), Applied and Environmental Microbiology 60:133-140 (1994)]. For the production of other than branched-chain amino acids, the strains bradytrophic for one of the branched-chain amino acids are used (Patent application WO01/09286 A1). Anabolic bradytroph (bradytrophy = slow nourishment) is a leaky auxotrophic mutant that is capable of growth in the absence of a supplement, but its growth rate is reduced because a function of an enzyme involved in biosynthesis of the supplement has been impaired. Bradytrophs are useful in elemination of feedback regulation of biosynthetic genes because if growth rate is limited by lack of anabolite, there will be no anabolite available for repression of the respective genes.

Valine producers of C. glutamicum are usually constructed by a deletion of the ilvA gene [Applied and Environmental Microbiology 68:2246-2250 (2002)]. Due to this deletion, no 2-ketobutyrate is formed in the cells of such strains and only pyruvate may serve as a substrate for the enzyme AHAS. The strains are thus isoleucine auxotrophs and this amino acid must be supplemented into the minimal growth medium. It was shown that production of valine is maximal under the conditions of isoleucine limitation. Under isoleucine shortage, transcriptional attenuation which controls the ilvBNC operon expression is partially relieved and activity of the enzymes coded by the genes of the operon is increased.

This invention describes strains bradytrophic for leucine and/or isoleucine, being superior in producing valine. The decrease of gene expression in P-panX, P-ilvA and P-leuA necessary therefore can be achieved by purposeful mutagenesis of the promoter's -10 hexamer motives.
Modifications of the promoters leading to the production of branched-chain amino acids can be constructed in the chromosome. The method for manipulations within the C. glutamicum chromosome (gene disruption and replacement) was described by Schwarzer and Pühler [Bio/Technology 9:84-87 (1991)]. General method for creating mutations, deletions, insertions and replacements using PCR was described by Horton [Molecular Biotechnology 3:93-99 (1995)]. The DNA fragments carrying the desired modifications are cloned in the E. coli vector which does not replicate in C. glutamicum such as pSUP301 [Bio/Technology 1:784-791 (1983)], or pK18mobsacB [Journal of Bacteriology 174:5462-5465 (1992)]. The vector constructs may be transferred to C. glutamicum by conjugation [Applied and Environmental Microbiology 60:756-759 (1994)]. In this regard it is referred to the new E. coli vector pKSAC45 (vector in E. coli DH5α deposited under DSM 18214), which is easily transferred to C. glutamicum by electrotransformation [FEMS Microbiology Letters 53:299-303 (1989); Example 1].

The weak mutant variants of promoters P-ilvA and P-leuA cause growth limitation of the respective strains. Growth limitation is thus introduced without necessity to supplement the strains by the required nutrient (isoleucine or leucine). In such fermentation the yield is maximized by growing the bradytrophic strain, which forms a limited concentration of a required nutrient without a possibility of overgrowth of the cells.

In addition to promoters with low activity, promoters P-ilvB, P-ilvD and P-ilvE are purposefully mutated to create promoters with higher activity. These stronger promoters of some genes may increase production of isoleucine and valine. By accumulation of the promoter modifications, strains with higher amino acid production are constructed. In addition to modify promoters, a mutation M13 of the enzyme AHAS (Feedback Resistant Mutants. European Patent Application No. 03014640.1) resulting in a feedback-resistant strain contributes to the increase of the amino acid's yield.

The promoters and recombinant DNA according to the invention are prepared by genetic engineering methods known to those skilled in the art (Sambrook et al. 1989. Molecular Cloning: A Laboratory Manual, 2nd edition, Cold Spring Harbor Laboratory Press; Balbas P. & Bolivar F. 1990, Design and construction of expression plasmid vectors in E. coli, Methods Enzymology 185, 14-37; Vectors: A Survey of Molecular Cloning Vectors and Their Uses. R.L. Rodriguez & D.T. Denhardt, eds: 205-225). As regards the general procedure (PCR, cloning, expression etc.), reference may also be made to the following literature and the material cited therein: Sambrook J., Fritsch E.F., Maniatis T. (1989). Molecular Cloning. Cold Spring Harbor Laboratory Press; Vectors: A Survey of Molecular Cloning Vectors and Their Uses. R.L. Rodriguez & D.T. Denhardt, II.
Procedure for improving the promoters according to the invention by mutagenesis methods are state of the art like e.g. saturation mutagenesis (A.R. Oliphant, A.L. Nussbaum, K. Struhl (1986) Cloning of random sequence oligonucleotides, Gene 44, 177-183), random mutagenesis (R.C. Caldwell, G.F. Joyce (1992) Randomization of genes by PCR mutagenesis, PCR Methods Appl. 2, 28-33), recombination methods such as shuffling (W.P. Stemmer (1994) DNA shuffling by random fragmentation and reassembly: In vitro recombination for molecular evolution, Proc. Natl. Acad. Sci. USA 91, 10747-10751), L-shuffling (EP 1104457) or StEP (H. Zhao, L. Giver, Z. Shao, J. Affholter, F. Arnold (1998) Molecular evolution by staggered extension process (StEP) in vitro recombination, Nat. Biotechnol. 16, 258-261), and site-directed mutagenesis (S.N. Ho, H.D. Hunt, R.M. Horton, J.K. Pullen, L.R. Pease (1989) Site-directed mutagenesis by overlap extension using the polymerase chain reaction, Gene 77, 248-254). The novel DNA sequences obtained are preferably transferred into phages, vectors or plasmids and further into host organism by the methods described above.

It is superfluous to note that among those modifications according to the invention further measures may be present in a microorganism for the production of branched-chain amino acids like for example: gene deletions, gene mutations, promoter insertions, deletions of regulatory sequences.
In particular it is preferred to construct micro-organisms having one or more modifications selected from the group consisting of ΔilvA, ΔpanB, PilvBMDEL, ilvNM13, PilvAM1CG, PilvDM14, PilvEM6, PleuAM3A, pECKAilvBNC in addition.

An exemplified general protocol for working the invention is given as follows: For genetic transfer of DNA fragments with modified promoters into Corynebacterium glutamicum cells and their vector-free integration into the chromosome, a vector replicating only in Escherichia coli is constructed. The 1387-bp fragment carrying the sacB gene is amplified by PCR using the oligonucleotide primers SACBF1 (SEQ. ID NO: 1) and SACBR1 (SEQ. ID NO: 2) with the target sites for the restriction enzyme BgIII appended to their 5' ends. The plasmid pK18mobsacB [Gene 145:69-73 (1994)] is used as a template. The BgIII-digested fragment is ligated with the vector pK45 [Applied Microbiology and Biotechnology 61, 523-527 (2003)] linearized also by BgIII. After ligation, E. coli DH5α cells are transformed with the ligation mixture and transformants containing the resulting plasmid pKSAC45 (vector in E. coli DH5α deposited under DSM 18214) are selected on agar plates containing kanamycin (20 mg/l). The identity of the resulting construct pKSAC45 is confirmed by restriction analysis, PCR and partial DNA sequencing. Its restriction and genetic map is shown in Fig. 2.

In order to reveal the actual key sequences of the promoters (promoter core sequences, i.e. -10 and -35 regions; see SEQ. ID NO.: 50-52) of the genes involved in branched-chain amino acid biosynthesis, which have not as yet been described (P-ilvN, P-ilvD and P-ilvE), the transcriptional start points (TSP) pertinent to the respective promoters are localised by primer extension analysis [Microbiology 142:1297-1309 (1996), Microbiology 144:915-927 (1998)] as follows: The DNA fragments carrying the promoter regions of the genes ilvN, ilvD and ilvE are cloned in the promoter-probe vector pET2 [Biotechnology Techniques 12:743-746 (1998)]. The resulting plasmids pETP-ilvN, pETP-ilvD and pETP-ilvE are transferred by electrotransformation into C. glutamicum ATCC13032. Total RNA from the strains C. glutamicum (pETP-ilvN), C. glutamicum (pETP-ilvD) and C. glutamicum (pETP-ilvE) is isolated using the hot phenol extraction [Molecular Microbiology 6:317-26 (1992)]. The primer extension analysis is carried out using the oligonucleotide primer CM4 (GAAAATCTCGTCGAAGCTCG) [Microbiology 142:1297-1309 (1996)] complementary to the sequence of the vector pET2 and using 30 µg total RNA. The reverse transcription and DNA electrophoresis is carried out as described [Microbiology 144:915-927 (1998)]. The acquired information on the core promoter sequences is utilized for the design of mutations in the promoters.

Construction of the modified ilvA promoters (P-ilvA) is performed using two successive PCR reactions and 4 oligonucleotide primers [Gene 102:67-70 (1991)].

The primers used:

| | | |
|---|---|---|
| ILVAIF3 | aggaattctagccgctcattttatgt | (SEQ. ID NO: 3) |
| ILVAIR2 | aagaattccgccaccgccga | (SEQ. ID NO: 4) |
| PILVAM1F | gagaagatcac(a/t)gtagtcaacc | (SEQ. ID NO: 5) |
| PILVAM1R | ggttgactac(t/a)gtgatcttctc | (SEQ. ID NO: 6) |
| PILVAM2F | gagaagattccag(t/a)agtcaacc | (SEQ. ID NO: 7) |
| PILVAM2R | ggttgact(a/t)ctggaatcttctc | (SEQ. ID NO: 8) |

First PCR: Using the primers ILVAIF3 and PILVAM1 R, the fragment A1 (760 bp) is amplified. Using the primers ILVAIF3 and PILVAM2R the fragment A2 (760 bp) is amplified. Using the primers PILVAM1F and ILVAIR2 the fragment 81 (646 bp) is amplified. Using the primers PILVAM2F and ILVAIR2 the fragment B2 (646 bp) is amplified. As a template, chromosomal DNA of C. glutamicum ATCC13032 is used in all cases. The resulting DNA fragments are separated in the agarose gel, isolated and purified by precipitation.

Second PCR: Using primers ILVAIF3 and ILVAIR2 and template fragments A1 + B1 or A2 + B2 (mixed in a molar ratio 1:1), a mixture 1 of fragments M1CG and M1CTG or mixture 2 of fragments M2CG and M2CGA (1384 bp) with mutations in the promoter P-ilvA are amplified.

These mixtures are digested by the restriction enzyme EcoRl and the resulting fragments are isolated from the agarose gel. The plasmid pKSAC45 is digested by EcoRI and ligated with the isolated fragments. Cells of E. coli DH5α are transformed with the ligation mixture and transformants are selected on the plates with kanamycin. Plasmid DNA is isolated from the resulting clones and the individual mutations within ilvA promoter are determined by sequencing. The mutant promoters P-ilvA M1CG (SEQ. ID NO: 32), P-ilvA M1CTG (SEQ. ID NO: 33), P-ilvA M2CG (SEQ. ID NO: 34), P-ilvA M2CGA (SEQ. ID NO: 35) are identified (wild type sequence: SEQ. ID NO: 31).

The resulting constructs of the vector pKSAC45 carrying the 1384-bp fragments with mutated promoters are isolated and transferred to C. glutamicum by electrotransformation [FEMS Microbiology Letters 53:299-303 (1989)]. Kanamycin-resistant clones were selected on the plates containing kanamycin.

The clones in which the double recombination event occurred are identified using the positive selection based on the conditional lethal effect of the sacB gene in C. glutamicum [Applied and Environmental Microbiology 60:756-759 (1994)]. The clones free of the vector sequences and carrying the mutations, are analysed by PCR and sequencing using the automatic sequencer ABI PRISM 3100 (Perkin Elmer).

Construction of the modified ilvB promoter (P-ilvB) is performed using PCR reactions and 4 oligonucleotide primers [Molecular Biotechnology 3:93-99 (1995)].

The primers used:

| | | |
|---|---|---|
| DELATTF | gtgaattcgacaacgatgccg | (SEQ. ID NO: 9) |
| DELATTR | gggaattccaccagaacagggcc | (SEQ. ID NO: 10) |
| ATT3 | caagccaagcgaataatggttatggtgtgtca | (SEQ. ID NO: 11) |
| ATT4 | accattattcgcttggcttggacgggtcc | (SEQ. 1D NO: 12) |

Transcription of the ilvBNC operon is regulated by the transcriptional attenuator, which controls the ilvB promoter [Journal of Bacteriology 175:5595-5603 (1993), Journal of Bioscience and Bioengineering 90:501-507 (2000)]. Using the designed oligonucleotide primers and two successive PCR reactions, a deletion of the attenuator (211 bp) is constructed in C. glutamicum ilvBNC operon.

First PCR: Using the primers DELATTF and ATT3 the fragment A (611 bp) is amplified. Using the primers ATT4 and DELATTR the fragment B (617 bp) is amplified. As a template, chromosomal DNA from C. glutamicum ATCC13032 is used in both cases. The resulting DNA fragments are separated in the agarose gel, isolated and purified by precipitation.

Second PCR: Using the primers DELATTF and DELATTR and template fragments A + B (mixed in a molar ratio 1:1), a fragment C (1218 bp) with the deleted ilvB attenuator is amplified. The fragment is digested by the restriction enzyme EcoRI and ligated with the plasmid pKSAC45 digested also by EcoRI. Cells of E. coli DH5α are transformed by the ligation mixture and transformants were selected on the plates with kanamycin.

Plasmid DNA is isolated from the resulting clones and the desired clone with the deletion within the ilvB attenuator (PilvBMDEL) iss identified by sequencing (SEQ. ID NO: 37, wild-type sequence: SEQ. ID NO: 36). The construct of pKSAC45 with modified ilvB promoter (P-ilvBDEL) is transferred to C. glutamicum by electrotransformation and the kanamycin-resistant clones are selected.

The clones in which the double recombination event occurred are identified using the positive selection based on the conditional lethal effect of the sacB gene in C. glutamicum. The clones free of the vector sequences and carrying the mutations, are analysed by PCR and sequencing using the automatic sequencer ABI PRISM 3100 (Perkin Elmer).

Construction of the modified ilvD promoters (P-ilvD) is performed using two successive PCR reactions and 4 oligonucleotide primers.

The primers used:

| | | |
|---|---|---|
| INSILVRF4 | tactgcagtctaaaatcatccacg | (SEQ. ID NO: 13) |
| INSILVDR | cgctgcaggagccacaggttgg | (SEQ. ID NO: 14) |
| PILVDM7F | tgcgtcactgtgctatagtaataag | (SEQ. ID NO: 15) |
| PILVDM7R | cttattacactatagcacagtgacgca | (SEQ. ID NO: 16) |
| PILVDM14F | tcaaagcactgtggtataataagcc | (SEQ. ID NO: 17) |
| PILVDM14R | ggcttattataccacagtgctttgacgca | (SEQ. ID NO: 18) |

First PCR: Using the primers INSILVDF4 and PILVDM7R, the fragment A7 (482 bp) is amplified. Using the primers INSILVDF4 and PILVDM14R the fragment A14 (484 bp) is amplified. Using the primers PILVDM7F and INSILVDR the fragment B7 (736 bp) is amplified. Using the primers PILVDM14F and INSILVDR the fragment B14 (732 bp) is amplified. As a template, chromosomal DNA of C. glutamicum ATCC13032 is used in all cases. The resulting DNA fragments are separated in the agarose gel, isolated and purified by precipitation.

Second PCR: Using primers INSILVDF4 and INSILVDR and template fragments A7 + B7 or A14 + B14 (mixed in a molar ratio 1:1), fragment named M7 or fragment named M14 (1191 bp) with mutations in the promoter P-ilvD were amplified.

These mixtures are digested by the restriction enzyme Pstl and the resulting fragments are isolated from the agarose gel. The plasmid pKSAC45 is digested PstI and ligated with the isolated fragments. Cells of E. coli DH5α are transformed with the ligation mixture and transformants are selected on the plates with kanamycin (30 mg/l). Plasmid DNA is isolated from the resulting clones and the individual mutations within ilvD promoter are determined by sequencing. The mutant promoters P-ilvDM7 (SEQ. ID NO: 39) and P-ilvDM14 (SEQ. ID NO: 40) are identified (wild type sequence: SEQ. ID NO: 38).

The resulting constructs of the vector pKSAC45 carrying the 1384-bp fragment with mutant promoters P-ilvDM7 or P-ilvDM14 are isolated and transferred to C. glutamicum by electrotransformation. Kanamycin-resistant clones were selected on the plates containing kanamycin.

The clones in which the double recombination event occurred are identified using the positive selection based on the conditional lethal effect of the sacB gene in C. glutamicum. The clones free of the vector sequences and carrying the mutations, are analysed by PCR and sequencing using the automatic sequencer ABI PRISM 3100 (Perkin Elmer).

Modified ilvE promoters (P-ilvE) are constructed using PCR reactions and 4 oligonucleotide primers.

The primers used:

| | | |
|---|---|---|
| PROILVEF | tcgaattctttgcgcagacccg | (SEQ. ID NO: 19) |
| PROILVER | ctgaattccaccggtgaagtaag | (SEQ. ID NO: 20) |
| PILVEM4F | gtagcaagtttaccttcaaatccatgacg | (SEQ. ID NO: 21) |
| PILVEM4R | cgtcatggatttgaaggtaaacttgctac | (SEQ. ID NO: 22) |
| PILVEM5F | gtagcaagtgtgccttaaaatccatgacg | (SEQ. ID NO: 23) |
| PILVEM5R | cgtcatggattttaaggcacacttgctac | (SEQ. ID NO: 24) |

First PCR: Using the primers PROILVEF and PILVEM4R the fragment A1 (677 bp) is amplified. Using the primers PROILVEF and PROILEM5R the fragment A2 (677 bp) is amplified. Using the primers PILVEM4F and PROILVER the fragment B1 (563 bp) is amplified. Using the primers PILVEM5F and PROILVER the fragment B2 (563 bp) is amplified. Chromosomal DNA of C. glutamicum ATCC13032 is used as a template in all cases. The resulting DNA fragments are separated in the agarose gel, isolated and purified by precipitation.

Second PCR: Using primers PROILVEF and PROILVER and template fragments A1 + B1 or A2 + B2 (mixed in a molar ratio 1:1), the fragments M4ATC or M5AG (1211 bp) with mutations in the promoter P-ilvE are amplified.

These mixtures were digested by the restriction enzyme EcoRI and the resulting fragments are isolated from the agarose gel. The plasmid pKSAC45 is digested EcoRI and ligated with the isolated fragments. Cells of E. coli DH5α are transformed with the ligation mixture and transformants are selected on the plates with kanamycin. Plasmid DNA is isolated from the resulting clones and the individual mutations within ilvE promoter are determined by sequencing. The mutant promoters P-ilvE M4ATC (SEQ. ID NO: 42), P-ilvE M5AG (SEQ. ID NO: 43) are identified (wild type sequence: SEQ. ID NO: 41).

The resulting constructs of the vector pKSAC45 carrying the 1207-bp fragment with mutated promoter are isolated and transferred to C. glutamicum by electrotransformation. Kanamycin-resistant clones are selected on the plates containing kanamycin.

The clones in which the double recombination event occurred are identified using the positive selection based on the conditional lethal effect of the sacB gene in C. glutamicum. The clones free of the vector sequences and carrying the mutations, are analysed by PCR and sequencing using the automatic sequencer ABI PRISM 3100 (Perkin Elmer).

Modified leuA promoters (P-leuA) are constructed using PCR reactions and 4 oligonucleotide primers.

The primers used are:

| | | |
|---|---|---|
| LEUAIF1 | ccgaattcgccgaactgaaatct | (SEQ. ID NO: 25) |
| LEUAIR1 | tcgaattctttgaagcccatctg | (SEQ. ID NO: 26) |
| PLEUAM2F | gttgtt(g/t)ca(t/g)gcttcaccacat | (SEQ. ID NO: 27) |
| PLEUAM2R | atgtggtgaagc(a/c)tg(c/a)aacaac | (SEQ. ID NO: 28) |
| PLEUAM3F | gttgttg(t/a)atgcatcaccacat | (SEQ. ID NO: 29) |
| PLEUAM3R | atgtggtgatgcat(a/t)caacaac | (SEQ. ID NO: 30) |

First PCR: Using the primers LEUAIF1 and PLEAM2R the fragment A1 (694 bp) is amplified. Using the primers LEUAIF1 and PLEUAM3R the fragment A2 (694 bp) is amplified. Using the primers PLEUAM2F and LEUAIRI the fragment B1 (472 bp) is amplified. Using the primers PLEUAM3F and LEUAIR1 the fragment B2 (472 bp) is amplified. As a template, chromosomal DNA of C. glutamicum ATCC13032 is used in all cases. The resulting DNA fragments are separated in the agarose gel, isolated and purified by precipitation.

Second PCR: Using the primers LEUAIF1 and LEUAIR1 and template fragments A1 + B1 or A2 + B2 (mixed in a molar ratio 1:1), mixture 1 of fragments 2C, 2CG and 2TCG or mixture 2 of fragments M3A and M3AA (1144 bp) with mutations in the promoter P-leuA are amplified.

These mixtures are digested by the restriction enzyme EcoRI and the resulting fragments were isolated from the agarose gel. The plasmid pKSAC45 is digested by EcoRI and ligated with the isolated fragments. Cells of E. coli DH5α are transformed by the ligation mixture and transformants were selected on the plates with kanamycin.

Plasmid DNA is isolated from the resulting clones and the individual mutations within leuA promoter are determined by sequencing. The mutant promoters P-leuA M2C (SEQ. ID NO: 45), M2CG (SEQ. ID NO: 46), P-leuA M2TCG (SEQ. ID NO: 47), P-leuA M3A (SEQ. ID NO: 48), P-leuA M3AA (SEQ. ID NO: 49) were identified (wild-type sequence: SEQ. ID NO: 44).

The constructs pKSAC46+promoter are transferred to C. glutamicum by electrotransformation and the kanamycin-resistant clones are selected. The clones in which the double recombination event occurred are identified using the positive selection based on the conditional lethal effect of the sacB gene in C. glutamicum. The clones free of the vector sequences and carrying the mutations, are analysed by PCR and sequencing using the automatic sequencer ABI PRISM 3100 (Perkin Elmer).

As can be seen from the appendant results, present strategy leads to superior valine and optionally isoleucine producer strains. It is noteworthy that more than 3-fold increases in fermentation property for these amino acids are achieved by merely changing the promoter sequences in an ingenious manner (ilvNM13 vs. itvNM13, PleuAM2TCG). This was neither anticipated nor made obvious by the prior art.

### Examples

### Example 1

L-valine production

Various strains obtained by introducing modified promoters and mutation ilvAM13 into the chromosome and the plasmid pECKAilvBNC into the cell of C. glutamicum were cultivated in the minimal medium CGXII [(Journal of Bacteriology 175:5595-5603, (1993)] and concentration of produced valine in the supernatant of the culture was determined in the following manner:

The strains were cultivated for 48 h in the medium CGXII (60 ml in 500-ml flasks, 30°C, 120 rpm). L-isoleucine (0-9 mM) and D-pantothenate (0.5 µM) were added to the cultures of strains with deletions ΔilvA and ΔpanB, respectively. Neither L-isoleucine nor L-leucine was added to the cultures of strains with leaky isoleucine or leucine phenotype. No supplements were added to the cultures of wild-type strain and of the strain with only mutation ilvNM13. Supernatant of 1ml aliquot, harvested from a culture, was used for determination of valine concentration. Valine concentration in the medium was determined by reversed-phase HPLC (HP1090; Hewlett-Packard, Waldbronn, Germany) with a fluorimetric detection (excitation at 230 nm, emission at 450 nm) after automatic precolumn derivatization with ortho-phthaldialdehyde. ODS Hypersil column was used (particle size 5 µm, 200×2.1 mm; Thermo Electron, USA). The buffer gradient consisted of a polar phase (0.05 M sodium acetate of pH 7.2) and non-polar phase (methanol). Quantification was done by calculation of valine concentration from calibration curve. The results are shown in Table 1.

**Table 1. Production of L-valine by the strains of Corynebacterium glutamicum**

| Strain of Corynebacterium glutamicum ATCC13032 | Production of L-valine (mM) |
|---|---|
| Wild type | 1 |
| ilvNM13 | 23 |
| ilvNM13 PilvEM6C | 27 |
| ilvNM13 PilvDM14 | 29 |
| ilvNM13 PilvDM7 | 35 |
| ilvNM13 PilvDM14 PilvEM6C | 32 |
| ilvNM13 PilvDM7 PilvEM6C | 38 |
| ilvNM13 PleuAM2C | 38 |
| ilvNM13 PleuAM2CG | 55 |
| ilvNM13 PleuAM2TCG | 77 |
| ilvNM13 PleuAM3A | 45 |
| ilvNM13 PleuAM3AA | 46 |
| ilvNM13 PilvAM1CTG | 40 |
| ilvNM13 PilvAM1CG | 40 |
| ΔilvA ΔpanB | 35 |
| ΔilvA ΔpanB ilvNM13 | 95 |
| ΔpanB ilvNM13 PilvAM1CG | 64 |
| ΔpanB ilvNM13 PilvAM1CG PilvDM14 PilvEM6C | 105 |
| ΔpanB ilvNM13 PiIvAMICG PilvDM7 PilvEM6C | 115 |
| ΔilvA ΔpanB ilvNM13 +plasmid pECKAilvBNC | 145 |
| ΔpanB ilvNM13 PiIvAM1CG PilvDM7 PilvEM6C +plasmid pECKAilvBNC | 162 |

## Claims

1. Process for the production of branched-chain amino acids by fermentation of a genetically modified bacterium of the genus Corynebacterium or Brevibacterium by attenuation of the promoter strength of one or more of the promoters selected from the group consisting of P-panX, P-ilvA and P-leuA and increasing the promoter strength of one or more of the promoters selected from the group consisting of P-ilvB, P-ilvC, P-ilvD and P-ilvE.

2. Process according to claim 1, wherein the branched-chain amino acid is valine.

3. Process according to claim 1 and/or claim 2, wherein the genetically modified bacterium is derived from a bacterium selected from the ATCC13032 and genetically related organisms like mutants of ATCC13032.

4. Process according to one or more of the preceding claims, wherein P-panX denotes promoters of panB- and/or panC-genes.

5. Process according to one or more of the preceding claims, wherein the promoter strength of P-ilvA and/or P-leuA is attenuated.

6. Process according to one or more of the preceding claims, wherein the -10-promoter-regions used for
a) ilvA are selected from the group consisting
of CACAGT, GACTGT;
b) ilvD are selected from the group consisting
of CACTGTGCTATAGTGTAAT, CAAAGCACTGTGGTATAAT;
c) ilvE are selected from the group consisting
of TGTGGTACAAT, TGTGGTACCAT
d) leuA are selected from the group consisting
of CATGCT, CAGGCT, CTGGCT, TATGCA, GAATGCA.

7. Promoter sequences for genes of Coryneform bacteria selected from the group consisting of ilvA, leuA, ilvD and ilvE comprising respective -10-motifs depicted in claim 6.

8. Recombinant DNA comprising promoter sequences of claim 7 and the respective genes selected from the group consisting of ilvA, leuA, ilvB, ilvD and ilvE.

9. Micro-organisms, plasmids, vectors and phages comprising the recombinant DNA of claim 8.

10. Use of a microorganism of claim 9 for the fermentative production of branched-chain amino acids.


SEQUENCE LISTING

<110> Degussa AG

<120> Promoter-Activity Modulation for Branched Chain-Amino Acid Production

<130> 200410045

<160> 52

<170> PatentIn version 3.1

<210> 1
<211> 24
<212> DNA
<213> Artificial

<220>
<223> Forward-primer sacB

<400> 1
gaagatctat cctttttaac ccat 24

<210> 2
<211> 24
<212> DNA
<213> Artificial

<220>
<223> Reverse-primer sacB

<400> 2
ggagatctcg ctcagtaccc atcg 24

<210> 3
<211> 26
<212> DNA
<213> Artificial

<220>
<223> Forward-primer ilvA

<400> 3
aggaattcta gccgctcatt ttatgt 26

<210> 4
<211> 20
<212> DNA
<213> Artificial

<220>
<223> Reverse-primer ilvA

<400> 4
aagaattccg ccaccgccga 20

<210> 5
<211> 22
<212> DNA
<213> Artificial

<220>
<223> Forward-primer P-ilvA1

<400> 5
gagaagatca cwgtagtcaa cc 22

<210> 6
<211> 22
<212> DNA
<213> Artificial

<220>
<223> Reverse-primer P-ilvA1

<400> 6
ggttgactac wgtgatcttc tc 22

<210> 7
<211> 22
<212> DNA
<213> Artificial

<220>
<223> Forward-primer P-ilvA2

<400> 7
gagaagattc cagwagtcaa cc 22

<210> 8
<211> 22
<212> DNA
<213> Artificial

<220>
<223> Reverse-primer P-ilvA2

<400> 8
ggttgactwc tggaatcttc tc 22

<210> 9
<211> 21
<212> DNA
<213> Artificial

<220>
<223> Forward-primer DELATT

<400> 9
gtgaattcga caacgatgcc g 21

<210> 10
<211> 23
<212> DNA

<213> Artificial

<220>
<223> Reverse-primer DELATT

<400> 10
gggaattcca ccagaacagg gcc 23

<210> 11
<211> 32
<212> DNA
<213> Artificial

<220>
<223> Primer ATT3

<400> 11
caagccaagc gaataatggt tatggtgtgt ca 32

<210> 12
<211> 29
<212> DNA
<213> Artificial

<220>
<223> Primer ATT4

<400> 12
accattattc gcttggcttg gacgggtcc 29

<210> 13
<211> 24
<212> DNA
<213> Artificial

<220>
<223> Primer

<400> 13
tactgcagtc taaaatcatc cacg 24

<210> 14
<211> 22
<212> DNA
<213> Artificial

<220>
<223> Primer

<400> 14
cgctgcagga gccacaggtt gg 22

<210> 15
<211> 25
<212> DNA
<213> Artificial

<220>
<223> Primer

<400> 15
tgcgtcactg tgctatagta ataag 25

<210> 16
<211> 27
<212> DNA
<213> Artificial

<220>
<223> Primer

<400> 16
cttattacac tatagcacag tgacgca 27

<210> 17

<211> 25
<212> DNA
<213> Artificial

<220>
<223> Primer

<400> 17
tcaaagcact gtggtataat aagcc 25

<210> 18
<211> 29
<212> DNA
<213> Artificial

<220>
<223> Primer

<400> 18
ggcttattat accacagtgc tttgacgca 29

<210> 19
<211> 22
<212> DNA
<213> Artificial

<220>
<223> Primer

<400> 19
tcgaattctt tgcgcagacc cg 22

<210> 20
<211> 23
<212> DNA
<213> Artificial

<220>

<223> Primer

<400> 20
ctgaattcca ccggtgaagt aag 23

<210> 21
<211> 29
<212> DNA
<213> Artificial

<220>
<223> Primer

<400> 21
gtagcaagtt taccttcaaa tccatgacg 29

<210> 22
<211> 29
<212> DNA
<213> Artificial

<220>
<223> Primer

<400> 22
cgtcatggat ttgaaggtaa acttgctac 29

<210> 23
<211> 29
<212> DNA
<213> Artificial

<220>
<223> Primer

<400> 23
gtagcaagtg tgccttaaaa tccatgacg 29

<210> 24
<211> 29
<212> DNA
<213> Artificial

<220>
<223> Primer

<400> 24
cgtcatggat tttaaggcac acttgctac 29

<210> 25
<211> 23
<212> DNA
<213> Artificial

<220>
<223> Primer

<400> 25
ccgaattcgc cgaactgaaa tct 23

<210> 26
<211> 23
<212> DNA
<213> Artificial

<220>
<223> Primer

<400> 26
tcgaattctt tgaagcccat ctg 23

<210> 27
<211> 22
<212> DNA
<213> Artificial

<220>
<223> Primer

<400> 27
gttgttkcak gcttcaccac at                                22

<210> 28
<211> 22
<212> DNA
<213> Artificial

<220>
<223> Primer

<400> 28
atgtggtgaa gcmtgmaaca ac                                22

<210> 29
<211> 22
<212> DNA
<213> Artificial

<220>
<223> Primer

<400> 29
gttgttgwat gcatcaccac at                                22

<210> 30
<211> 22
<212> DNA
<213> Artificial

<220>
<223> Primer

<400> 30

atgtggtgat gcatwcaaca ac                                    22

<210> 31
<211> 23
<212> DNA
<213> coryneform bacterium

<220>
<221> misc_feature
<222> (9)..(14)
<223> -10 hexamer

<400> 31
gagaagatta cactagtcaa cca                                   23

<210> 32
<211> 23
<212> DNA
<213> Artificial

<220>
<223> Primer

<220>
<221> misc_feature
<222> (9)..(14)
<223> -10 hexamer

<400> 32
gagaagatca cagtagtcaa cca                                   23

<210> 33
<211> 23
<212> DNA
<213> Artificial

<220>
<223> Primer

<220>
<221> misc_feature
<222> (9)..(14)
<223> -10 hexamer

<400> 33
gagaagatca ctgtagtcaa cca 23

<210> 34
<211> 23
<212> DNA
<213> Artificial

<220>
<223> Primer

<220>
<221> misc_feature
<222> (9)..(14)
<223> -10 hexamer

<400> 34
gagaagattc cagtagtcaa cca 23

<210> 35
<211> 23
<212> DNA
<213> Artificial

<220>
<223> Primer

<220>
<221> misc_feature

<222> (9)..(14)
<223> -10 hexamer

<400> 35
gagaagattc cagaagtcaa cca                                    23

<210> 36
<211> 379
<212> DNA
<213> Artificial

<220>
<223> Sequence

<220>
<221> -35_signal
<222> (50)..(55)
<223>

<220>
<221> misc_structure
<222> (377)..(379)
<223> Start ilvB

<220>
<221> -10_signal
<222> (63)..(68)
<223>

<400> 36
tccttaaccg aagtggggga gttttggggg tgggaatttt cgtgcgttgt ggaattggaa    60

actcgatgtg tgtagcatga cacaccatga ccattattcg acttgtagta gtaaccgcgc    120

ggcgcctgcc gtaacggcct tccaagtcgt ctcgtcaagc gccctcgaca acactcacca    180

cagtgttgga acgagggctt tcttgttggt tatgacccaa gtagccaact ttgcaacaga    240

catctgtcgc actgcgtgca cagcatccgc gtcggaacaa ttttaaatga gggctttgtc 300

tttaggctga gttgaaatcg gcttggcttg gacgggtcct gtgaaaatcc ttatttagta 360

aaggagccag aaagtcgtg 379

<210> 37
<211> 159
<212> DNA
<213> Artificial

<220>
<223> Sequence

<220>
<221> -35_signal
<222> (50)..(55)
<223>

<220>
<221> misc_structure
<222> (157)..(159)
<223> Start ilvB

<220>
<221> -10_signal
<222> (73)..(78)
<223>

<400> 37
tccttaaccg aagtggggga gttttggggg tgggaatttt cgtgcgttgt ggaattggaa 60

actcgatgtg tgtagcatga cacaccataa ccattattcg gcttggcttg gacgggtcct 120

gtgaaaatcc ttatttagta aaggagccag aaagtcgtg 159

<210> 38
<211> 41

<212> DNA
<213> coryneform bacterium

<220>
<221> -10_signal
<222> (24)..(29)
<223>

<220>
<221> -10_signal
<222> (29)..(34)
<223>

<400> 38
gtgataaccc tgcgtcacag cactagagtg taataagccg t 41

<210> 39
<211> 41
<212> DNA
<213> Artificial

<220>
<223> Primer

<220>
<221> -10_signal
<222> (24)..(29)
<223>

<220>
<221> -10_signal
<222> (29)..(34)
<223>

<400> 39
gtgataaccc tgcgtcactg tgctatagtg taataagccg t 41

<210> 40
<211> 41

<212> DNA
<213> Artificial

<220>
<223> Primer

<220>
<221> -10_signal
<222> (24)..(29)
<223>

<220>
<221> -10_signal
<222> (29)..(34)
<223>

<400> 40
gtgataaccc tgcgtcaaag cactgtggta taataagccg t 41

<210> 41
<211> 31
<212> DNA
<213> coryneform bacterium

<220>
<221> -10_signal
<222> (18)..(23)
<223>

<400> 41
ctgtcaggta gcaggtgtac cttaaaatcc a 31

<210> 42
<211> 31
<212> DNA
<213> Artificial

<220>
<223> Primer

<220>
<221> -10_signal
<222> (18)..(23)
<223>

<400> 42
ctgtcaggta gcaagtttac ctcaaaatcc a 31

<210> 43
<211> 31
<212> DNA
<213> Artificial

<220>
<223> Primer

<220>
<221> -10_signal
<222> (18)..(23)
<223>

<400> 43
ctgtcaggta gcaagtgtgc cttaaaatcc a 31

<210> 44
<211> 26
<212> DNA
<213> coryneform bacterium

<220>
<221> -10_signal
<222> (13)..(18)
<223>

<400> 44
acaaagttgt tgtatgcttc accaca 26

<210> 45
<211> 26
<212> DNA
<213> Artificial

<220>
<223> Primer

<220>
<221> -10_signal
<222> (13)..(18)
<223>

<400> 45
acaaagttgt tgcatgcttc accaca 26

<210> 46
<211> 26
<212> DNA
<213> Artificial

<220>
<223> Primer

<220>
<221> -10_signal
<222> (13)..(18)
<223>

<400> 46
acaaagttgt tgcaggcttc accaca 26

<210> 47
<211> 26
<212> DNA
<213> Artificial

<220>
<223> Primer

<220>
<221> -10_signal
<222> (13)..(18)
<223>

<400> 47
acaaagttgt ttctggcttc accaca 26

<210> 48
<211> 26
<212> DNA
<213> Artificial

<220>
<223> Primer

<220>
<221> -10_signal
<222> (13)..(18)
<223>

<400> 48
acaaagttgt tgtatgcatc accaca 26

<210> 49
<211> 26
<212> DNA
<213> Artificial

<220>
<223> Primer

<220>
<221> -10_signal
<222> (13)..(18)
<223>

<400> 49

acaaagttgt tgaatgcatc accaca                                26

<210>  50
<211>  120
<212>  DNA
<213>  coryneform bacterium

<220>
<221>  -35_signal
<222>  (81)..(86)
<223>

<220>
<221>  misc_structure
<222>  (115)..(115)
<223>  Transcriptional Start Point

<220>
<221>  -10_signal
<222>  (103)..(108)
<223>

<400>  50
agttgaaggt ttccccatta agatcgcact aatcaacaac ggaaacctgg gcatggttcg    60

ccaatggcag accctattct atgaaggacg gtactcaaat actaaacttc gtaaccaggg    120

<210>  51
<211>  120
<212>  DNA
<213>  coryneform bacterium

<220>
<221>  -35_signal
<222>  (81)..(85)
<223>

<220>

<221> -10_signal
<222> (100)..(105)
<223>

<220>
<221> -10_signal
<222> (105)..(110)
<223> -10 hexamer to alternative TSP

<220>
<221> -35_signal
<222> (86)..(91)
<223> -35 hexamer to alternative TSP

<400> 51
accgatggga atacaacaca aacagaaagc gttgaatccc aagagaccgg acaagccgcg 60

tctgaaacct cacatcgtga taaccctgcg tcacagcact agagtgtaat aagccgtccg 120

<210> 52
<211> 120
<212> DNA
<213> coryneform bacterium

<220>
<221> -35_signal
<222> (65)..(70)
<223>

<220>
<221> -10_signal
<222> (88)..(93)
<223>

<400> 52
cctagccatt cctcaaaacc gtgagacgaa attggctatt catcccataa aatggggctg 60

actagtgtat ctgtcaggta gcaggtgtac cttaaaatcc atgacgtcat tagagttcac 120